# EUROPÄISCHE PATENTANMELDUNG

(11) **EP 3 257 472 A1**
(43) Veröffentlichungstag der Anmeldung: **20.12.2017**
(21) Anmeldenummer: 17174230.7
(22) Anmeldetag: 02.06.2017
(51) Int. Cl.: A61F 2/24

(54) **HERZKLAPPENPROTHESE MIT AUSSENSCHÜRZE**

(30) Priorität: 17.06.2016 DE 102016111103
(71) Anmelder: Biotronik AG, 8180 Bülach (CH)
(72) Erfinder: MAYER, Arnulf, 8180 Bülach (CH)
(74) Vertreter: Randoll, Sören

(57) **Zusammenfassung**

Die Erfindung betrifft eine Herzklappenprothese (1), aufweisend eine Herzklappe (2), einen in einer Umfangsrichtung (U) umlaufenden Grundkörper (3) zum Tragen der Herzklappe (2), die an dem Grundkörper (3) befestigt ist.

## Beschreibung

Eine derartige Herzklappenprothese weist eine Herzklappe auf, die eine Herzklappe des Patienten ersetzen soll, sowie einen Grundkörper, bevorzugt in Form eines Stents, der zum Tragen der Ersatzherzklappe dient, wobei jene Ersatzherzklappe an dem Grundkörper befestigt ist. Ferner umfasst die Herzklappenprothese eine Schürzenanordnung, die geeignet ist paravalvuläre Leckagen abzudichten.

Herzklappenprothese werden z.B. im Rahmen einer Transkatheter-Aortenklappenimplantation verwendet (auch TAVI genannt, für transcatheter aortic valve implantation). Hierbei stellt die paravalvuläre Leckage, bei der Blut zwischen der Gefäßwand und der Prothese hindurch strömt, eine der schwerwiegenden Komplikationen dar, die die prozedurale Effektivität minimalinvasiver Herzklappen beschränkt.

Aus dem Stand der Technik sind diesbezüglich Abdichtungsmaßnahmen bekannt. So wird z. B. gemäß EP 2 749 254 A1 eine Gewebedichtung vorgeschlagen, bei der eine Gewebedichtung von einem distalen Ende der Herzklappe sich in proximaler Richtung zurück über einen Anker der Herzklappe erstreckt, wobei die Gewebedichtung im expandierten Zustand der Prothese in Falten legt und Gewebelaschen und -taschen bildet, die Blut aufnehmen können und somit eine Dichtwirkung entfalten können. Jedoch ist diese Anordnung nicht dazu geeignet, insbesondere bei einer Diastole sich gerichtet und effektiv mit Blut zu füllen, um paravalvuläre Leckagen zu schließen, wenn die künstliche Herzklappe geschlossen ist.

Hiervon ausgehend liegt der vorliegenden Erfindung die Aufgabe zugrunde, eine Herzklappenprothese zu schaffen, die dem Risiko einer paravalvulären Leckage entgegen wirkt und insbesondere eine zielgerichtete und verbesserte Abdichtung bereitstellt.

Diese Aufgabe wird durch eine Herzklappenprothese mit den Merkmalen des Anspruchs 1 gelöst. Vorteilhafte Ausgestaltungen der Erfindung sind in den Unteransprüchen angegeben und werden nachfolgend beschrieben.

Gemäß Anspruch 1 wird eine Herzklappenprothese vorgeschlagen, mit einer Herzklappe, einen in einer Umfangsrichtung umlaufenden Grundkörper zum Tragen der Herzklappe, die an dem Grundkörper befestigt ist, wobei der Grundkörper einen Innenraum umgibt und eine dem Innenraum zugewandte Innenseite sowie eine dem Innenraum abgewandte Außenseite aufweist, wobei die Herzklappe in dem Innenraum angeordnet ist, und wobei die Herzklappenprothese eine im Innenraum angeordnete Innenschürze aufweist, die an einer Innenseite des Grundkörpers befestigt ist und in der Umfangsrichtung des Grundkörpers umläuft, wobei die Innenschürze bevorzugt einen Einströmbereich des Grundkörpers auskleidet, über den Blut in den Innenraum hin zur Herzklappe einströmen kann.

Erfindungsgemäß ist nun vorgesehen, dass die Herzklappenprothese zur Vermeidung einer paravalvulären Leckage der Herzklappenprothese des Weiteren eine auf der Außenseite des Grundkörpers angeordnete (vorzugsweise separate) Außenschürze aufweist, die den Grundkörper umgibt (vorzugsweise umgibt die Außenschürze den Einströmbereich des Grundkörpers von außen her, so dass der entsprechende Abschnitt des Grundkörpers zwischen der Innenschürze und der Außenschürze angeordnet ist) und in der Umfangsrichtung umläuft, wobei die beiden Schürzen eine Aufnahme zum Aufnehmen von Blut ausbilden, die dazu konfiguriert ist, während der Diastole Blut aufzunehmen und anzusammeln, so dass sich die Außenschürze dauerhaft in Richtung auf die Herzklappenprothese umgebendes Gewebe des Patienten ausbaucht und abdichtend an dieses anlegt. Hierdurch wird eine paravalvuläre Leckage wirksam unterdrückt bzw. ein derartiges Risiko signifikant verringert.

Vorzugsweise handelt es sich bei dem besagten Gewebe um den Anulus einer nativen (defekten bzw. zu ersetzenden) Aortenklappe oder zumindest einen Abschnitt des linksventrikulären Ausflusstrakts (LVOT). Die Abdichtung findet also bevorzugt am Anulus bzw. am LVOT statt.

Gemäß einer bevorzugten Ausführungsform der erfindungsgemäßen Herzklappenprothese ist vorgesehen, dass sich der Grundkörper in einer axialen Richtung ausgehend von einem Einströmbereich (siehe oben), über den Blut in den Innenraum des Grundkörpers in einer Strömungsrichtung bzw. in der axialen Richtung einströmen kann, zu einem Ausströmbereich hin erstreckt, über den die Herzklappe passierendes Blut in der Strömungsrichtung bzw. in der axialen Richtung aus dem Innenraum des Grundkörpers austreten kann.

Weiterhin ist gemäß einer bevorzugten Ausführungsform der erfindungsgemäßen Herzklappenprothese vorgesehen, dass die Außenschürze einen ersten (insbesondere umlaufenden) Randbereich sowie einen zweiten (insbesondere umlaufenden) Randbereich aufweist, wobei die beiden Randbereiche über einen mittleren Abschnitt der Außenschürze miteinander (z. B. einstückig) verbunden sind.

Diesbezüglich ist gemäß einer Ausführungsform bevorzugt vorgesehen, dass der erste Randbereich der Außenschürze an einem den Einströmbereich des Grundkörpers begrenzenden Randbereich des Grundkörpers befestigt ist, insbesondere festgenäht ist. Es ist dabei insbesondere bevorzugt, dass der erste Randbereich der Außenschürze im Einströmbereich an den Grundkörper so befestigt, bevorzugt festgenäht ist, dass dieser dicht, insbesondere fließdicht mit der im Einströmbereich innen befestigen Innenschürze abschließt. Somit wird vorteilhaft gewährleistet, dass sich Blut während der Diastole im Zwischenbereich vom Außenschürze und Innenschürze ansammeln kann und die Menge angesammelten Bluts mit jeder Diastole zunimmt bis der Zwischenbereich druckvoll und umfänglich mit Blut gefüllt ist.

Weiterhin ist diesbezüglich gemäß einer Ausführungsform bevorzugt vorgesehen, dass der mittlere Abschnitt der Außenschürze nicht oder lediglich lose am Grundkörper befestigt ist, so dass sich der mittlere Abschnitt zu dem Gewebe hin ausbauchen kann. Dies gilt insbesondere sowohl im komprimierten Zustand des Grundkörpers des (zum Beispiel wenn dieser mittels eines Katheters zum Implantationsort transportiert wird), als auch im expandierten sowie implantierten Zustand das Grundkörpers.

Weiterhin ist gemäß einer bevorzugten Ausführungsform vorgesehen, dass der zweite Randbereich der Außenschürze am Ausströmbereich des Grundkörpers befestigt ist, insbesondere festgenäht ist, oder zumindest benachbart zum Austrittbereich/Ausströmbereich des Grundkörpers an diesem befestigt ist, insbesondere festgenäht ist.

Weiterhin ist gemäß einer Ausführungsform der erfindungsgemäßen Herzklappenprothese vorgesehen, dass die Innenschürze einen ersten (vorzugsweise umlaufenden) Randbereich sowie einen dem ersten Randbereich in der axialen Richtung gegenüberliegenden zweiten (vorzugsweise umlaufenden) Randbereich aufweist, wobei der erste Randbereich der Innenschürze bevorzugt an dem den Einströmbereich des Grundkörpers begrenzenden Randbereich befestigt ist, insbesondere festgenäht ist, und wobei der zweite Randbereich der Innenschürze bevorzugt näher am Ausströmbereich gelegen am Grundkörper befestigt ist.

Weiterhin ist gemäß einer Ausführungsform hierbei bevorzugt vorgesehen, dass der zweite Randbereich der Außenschürze in der axialen Richtung über den zweiten Randbereich der Innenschürze verlängert ist und/oder hinaus ragt, wobei der zweite Randbereich der Außenschürze vorzugsweise in der axialen Richtung nicht über die Kommissuren der Herzklappe hinaus ragt. Eine solche Anordnung liegt insbesondere bevorzugt im expandierten Zustand vor, wenn die Herzklappenprothese implantiert ist.

Auf diese Weise bilden gemäß einer Ausführungsform der erfindungsgemäßen Herzklappenprothese der zweite Randbereich der Außenschürze und der zweite Randbereich der Innenschürze eine in der Umfangsrichtung umlaufende Einströmöffnung der besagten Aufnahme, wobei diese Einströmöffnung dem Innenraum des Grundkörpers zugewandt ist. Während der jeweiligen Diastole kann somit leicht über die Einströmöffnung Blut in die Aufnahme einfließen und sich dort Herzschlag für Herzschlag ansammeln, wobei sich eine gegenüber dem Gewebe abdichtende Wirkung der Außenschürze einstellt, die mit zunehmendem Füllungsgrad der Aufnahme stärker gegen den umgebenden Anulus bzw. LVOT gedrückt wird. Durch die Anordnung der Außenschürze bezüglich der Innenschürze vergrößert sich der Querschnitt der umlaufenden Einstellungsöffnung bevorzugt während der Diastole, was ein Einströmen von Blut in die Aufnahme begünstigt.

In einer bevorzugten Ausführungsform ist die Einströmöffnung direkt oberhalb der künstlichen Klappensegel angeordnet. Oberhalb der Klappensegel soll hier bedeuten, dass die Einströmöffnung in Blutflussrichtung hinter den Klappensegeln angeordnet ist. Der Grundkörper ist in seinen Maßen so ausgestaltet, dass die Klappenprothese in der Weise in der alten natürlichen Herzklappe zum Liegen kommt, dass die Klappensegel der Herzprothese sich in einem Bereich des Blutgefäßes befinden, der einen sehr geringen Durchmesser aufweist und im Vergleich zum umliegenden Gewebe sehr eng ist. Im Falle einer Herzklappenprothese für die Aortenklappe (Aortenherzklappenprothese) ist die Einströmöffnung im Bereich der Sinusse angeordnet, wodurch diese sehr breit wird. Dies ist vorteilhaft, da erreicht wird, dass beim Blutrückfluss, beispielsweise während einer Diastole im Falle einer Aortenherzklappenprothese, der Fluss des Blutes über die Herzklappensegel zielgerichtet und mit erhöhtem Druck in die weite Einströmöffnung einfließt, wodurch die Ausbauchung der Außenschürze mit größter Kraft und hohem Blutvolumen vollzogen wird. Hindurch werden paravalvuläre Leckagen mit höchster Effektivität unterbunden. Ferner wird durch die Einpressung von Blut unter erhöhtem Druck die Bildung von stationären Thromben, die sich in dem Zwischenraum zwischen Innen- und Außenschürze bilden und paravalvuläre Leckagen nachhaltig und endgültig abdichten, unterstützt.

Bevorzugt handelt es sich bei dem besagten Grundkörper um einen sogenannten Stent, der eine Mehrzahl an Streben aufweist, die eine Zellstruktur ausbilden. Diese ist durch eine Mehrzahl an Zellen gekennzeichnet, bei denen es sich umso genannte offene oder geschlossene Zellen handeln kann.

Vorzugsweise ist weiterhin vorgesehen, dass der Grundkörper der Prothese dazu konfiguriert ist, expandiert zu werden (z. B. in bekannter Weise durch einen Ballon), um insbesondere die Herzklappenprothese am Implantationsort in einen bestimmungsgemäßen Zustand zu expandieren.

Gemäß einer alternativen Ausführungsform der Erfindung ist vorgesehen, dass der Grundkörper selbstexpandierend ausgebildet ist. Hier ist der Grundkörper vorzugsweise aus einem Material geschaffen, das eine automatische Expansion des Grundkörpers ermöglicht, sobald dieser entsprechend freigegeben wird (z.B. aufgrund einer elastischen, insbesondere superelastischen Eigenschaft des Grundkörpers).

Besonders bevorzugt ist die Herzklappenprothese dazu konfiguriert, per Katheter in ein Körperlumen eines Patienten implantiert zu werden, vorzugsweise mittels TAVI. Entsprechend betrifft ein weiterer Aspekt der Erfindung ein System mit einer erfindungsgemäßen Herzklappenprothese und einer Kathetereinrichtung, die zum Implantieren der Herzklappenprothese konfiguriert ist.

Weiterhin ist gemäß einer Ausführungsform der Erfindung vorgesehen, dass die Herzklappe ein biologisches Gewebe, insbesondere Perikard, aufweist.

Weiterhin ist gemäß einer Ausführungsform der erfindungsgemäßen Herzklappenprothese vorgesehen, dass die künstliche Herzklappe, die Außenschürze und/oder die Außenschürze eines der folgenden Materialen aufweist oder aus einem der folgenden Materialien besteht: mikrobielle Cellulose, oder ferner grundsätzlich alle Arten von tierischem Gewebe, insbesondere Gewebe vom Säugetier, einschließlich vom Menschen. Bevorzugt ist nichthumanes Gewebe. Insbesondere eignen sich solche Gewebe, die als Klappenmaterial in einer Herzklappe verwendet werden können. Bevorzugt sind dabei Perikardgewebe und Herzklappen, aber auch Haut, Bandgewebe, Sehnen, Bauchfell, Dura mater, Tela submucosa, insbesondere des Magen-Darm-Traktes, oder Rippenfell. Bei Herzklappen können alle Klappen verwendet werden, also Aorten-, Pulmonal-, Mitral- und Trikuspidalklappen. Des Weiteren sind bevorzugt Perikardgewebe vom Schwein, Schaf, Ziege, Pferd, Krokodil, Känguru, Strauß und vom Rind. Herzklappe, Innenschürze und Außenschürze können dabei aus demselben Material oder aus unterschiedlichen Materialien hergestellt sein.

Weiterhin können insbesondere Außen- und Innenschürze auch aus künstlichem polymerem Material gebildet sein oder ein solches umfassen. In einer bevorzugten Ausführungsform sind Außen- und/oder Innenschürze aus Polyether, Polyolefin, insbesondere Polyethylen, Polypropylen und Polytetrafluoroethylen, oder Polyester, insbesondere Dacron^{®} ausgebildet. In einer speziellen Ausführungsform ist die Außenschürze aus einem der oben genannten künstlichen polymeren Materialien gebildet, insbesondere Polytetrafluoroethylen oder Dacron^{®}. In einer weiteren Ausführungsform kann die Außenschürze aus einem nachgiebigen oder elastischen Material hergestellt sein, durch das es ermöglicht wird, Bereiche, in denen paravalvuläre Leckagen auftreten, durch erhöhtem Druck in der Aufnahme zwischen Außen- und Innenschürze, besser auszukleiden und damit abzudichten.

Besonders bevorzugt ist vorgesehen, dass die Herzklappe dazu ausgebildet ist, eine defekte native Aortenklappe zu ersetzen.

Weitere Merkmale und Vorteile der Erfindung sollen nachfolgend anhand von Figurenbeschreibungen von Ausführungsformen der Erfindung beschrieben werden. Es zeigt:

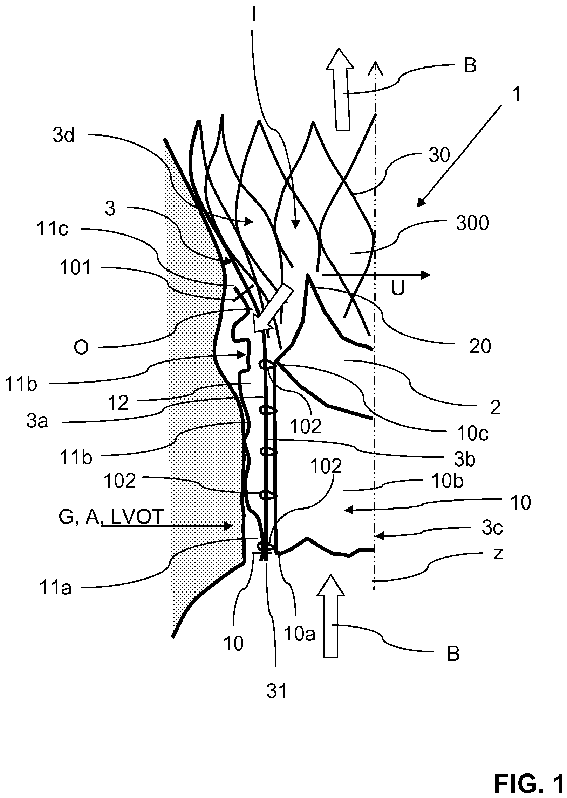

- Fig. 1: eine schematische teilweise geschnittene Ansicht einer erfindungsgemäßen Herzklappenprothese.

Gemäß Figur 1 weist die Herzklappenprothese 1 mit einer vorzugsweise künstlichen Herzklappe 2 aus einem biologischen Gewebe mit Kommissuren 20 auf und einen Grundkörper 3, vorzugsweise in Form eines expandierbaren oder selbstexpandierenden Stents (siehe auch oben), der dazu konfiguriert ist, die Herzklappe 2 in einem vom Grundkörper 3 umgebenen Innenraum I aufzunehmen, wobei die Herzklappe 2 an dem Grundkörper 3 befestigt ist, z.B. durch Vernähen der Herzklappe 2 mit dem Grundkörper 3. Der Grundkörper 3 weist vorzugsweise eine Mehrzahl an Streben 30 auf, die eine Zellstruktur mit z.B. geschlossenen Zellen 300 ausbilden. Die Streben 30 sind in der Fig. 1 nur bereichsweise dargestellt, so dass die Schürze 10 aus Gründen der Übersichtlichkeit nicht verdeckt ist.

Im implantierten und expandierten Zustand kann die Herzklappenprothese 1 z.B. dazu konfiguriert sein, eine defekte Aortenklappe eines Patienten zu ersetzen bzw. zu verdrängen. Das Blut strömt dann in der Strömungsrichtung B durch die am expandierten Grundkörper 3 befestigte Ersatzherzklappe 2, wobei die Prothese 1 umlaufend an umgebenden Gewebe G bzw. der umgebenden Gefäßwand G abdichtend anliegen soll, wobei bevorzugt eine Abdichtung im Bereich des Anulus A der defekten nativen Herzklappe bzw. am linksventrikulären Ausströmtrakt LVOT erfolgt, um einen Blutfluss zwischen dem Gewebe bzw. der Gefäßwand G und der Prothese 1 zu vermeiden. Ein solcher Blutfluss stellt eine sogenannte paravalvuläre Leckage dar.

Die Herzklappenprothese 1 weist nun erfindungsgemäß zum Verhindern einer solchen paravalvulären Leckage eine im Innenraum I angeordnete Innenschürze 10 auf, die an einer Innenseite 3b des Grundkörpers 3 befestigt ist und in der Umfangsrichtung U des Grundkörpers 3 umläuft, sowie des Weiteren eine auf der Außenseite 3a des Grundkörpers 3 angeordnete Außenschürze 11, die den Grundkörper 3 umgibt und in der Umfangsrichtung U umläuft, wobei die beiden Schürzen 10, 11 eine Aufnahme 12 zum Aufnehmen von Blut ausbilden, die dazu konfiguriert ist, während der Diastole Blut aufzunehmen und anzusammeln, so dass sich die Außenschürze 11 in Richtung auf die Herzklappenprothese 1 umgebendes Gewebe G des Patienten bzw. in Richtung auf den Anulus A oder den LVOT ausbaucht und abdichtend an diese Gefäßstrukturen anlegt. Die Aufnahme 12 stellt somit den axial und in Umfangsrichtung U verlaufenden Zwischenraum zwischen Außen- und Innenschürze dar, der durch die feste Verbindung beider Schürzen im Einströmbereich begrenzt ist.

Im Einzelnen ist hierbei insbesondere vorgesehen, dass sich der Grundkörper 3 in einer axialen Richtung z ausgehend von einem Einströmbereich 3c des Grundkörpers 3 bzw. der Prothese 1, über den Blut in den Innenraum I in einer Strömungsrichtung B einströmen kann, zu einem Ausströmbereich 3d des Grundkörpers 3 bzw. der Prothese 1 hin erstreckt, über den die Herzklappe 2 passierendes Blut in der Strömungsrichtung B aus dem Innenraum (I) austreten kann.

Die Außenschürze 11 weist gemäß Figur 1 einen ersten in der Umfangsrichtung U umlaufenden Randbereich 11a sowie einen zweiten im der Umfangsrichtung U umlaufenden Randbereich 11c auf, wobei die beiden Randbereiche 11a, 11c über einen mittleren Abschnitt 11b der Außenschürze 11 beispielsweise und bevorzugt einstückig miteinander verbunden sind. Hierbei ist der erste Randbereich 11a der Außenschürze 11 an einem den Einströmbereich 3c begrenzenden Randbereich 31 des Grundkörpers 3 befestigt, z.B. mittels geeigneter Nähte 100 oder einem sonstigen geeigneten Verbindungsmittel 100. Weiterhin ist der zweite Randbereich 11c der Außenschürze 11 am Ausströmbereich 3d des Grundkörpers 3 befestigt, z.B. ebenfalls mittels Nähten 101 oder einem sonstigen Verbindungsmittel 101. Im Gegensatz zu den beiden Randbereichen 11a, 11c ist der mittlere Abschnitt 11b der Außenschürze nicht oder lediglich locker am Grundkörper 3 befestigt, so dass dieser eine lose Materiallage bildet, die sich nach außen hin zu Dichtungszwecken ausbeulen kann, wenn sich die Aufnahme 12 mit Blut füllt.

An der Innenseite 3a des Grundkörpers 3 wird die Aufnahme 12 durch die Innenschürze 10 begrenzt, die in der axialen Richtung z aufeinanderfolgend einen ersten Randbereich 10a, dann einen mittleren Abschnitt 10b und nachfolgend einen zweiten Randbereich 10c aufweist, wobei 10b den Einströmbereich des Grundkörpers 3 bzw. der Prothese 1 auskleidet.

Der erste Randbereich 10a der Innenschürze 10 ist wiederum an dem den Einströmbereich 3c des Grundkörpers 3 begrenzenden Randbereich 31 befestigt, wozu z.B. Nähte 102 oder ein sonstiges geeignetes Verbindungsmittel 102 verwendet werden können. Der zweite Randbereich 10c der Innenschürze 10 ist demgegenüber näher am Ausströmbereich 3d gelegen und dort am Grundkörper 3 befestigt z.B. mittels Nähten 102 oder einem sonstigen geeigneten Verbindungsmittel 102.

Besonders bevorzugt ist vorgesehen, dass der zweite Randbereich 11c der Außenschürze 11 in der axialen Richtung z über den zweiten Randbereich 10c der Innenschürze 10 hinaus erstreckt ist. Ferner ist bevorzugt, dass der zweite Randbereich 11c der Außenschürze 11 in der axialen Richtung z nicht über die Kommissuren 20 der Herzklappe 2 hinaus steht. Eine solche Anordnung liegt insbesondere im expandierten Zustand vor, wenn die Herzklappenprothese implantiert ist.

Der zweite Randbereich 11c der Außenschürze 11 und der zweite Randbereich 10c der Innenschürze 10 bilden auf diese Weise eine in der Umfangsrichtung U umlaufende Einströmöffnung O der Aufnahme 12 über die das Blut in der Diastole in die Aufnahme 12 gelangt und sich dort ansammelt, wobei sich die abdichtende Wirkung der Außenschürze 11 einstellt.

## Patentansprüche

1. Herzklappenprothese (1), aufweisend eine Herzklappe (2), einen in einer Umfangsrichtung (U) umlaufenden Grundkörper (3) zum Tragen der Herzklappe (2), die an dem Grundkörper (3) befestigt ist, wobei der Grundkörper (3) einen Innenraum (I) der Herzklappenprothese (1) umgibt, wobei die Herzklappe (2) in dem Innenraum (I) angeordnet ist, und wobei die Herzklappenprothese (1) eine im Innenraum (I) angeordnete Innenschürze (10) aufweist, die an der Innenseite (3b) des Grundkörpers (3) befestigt ist und in der Umfangsrichtung (U) des Grundkörpers (3) umläuft,
**dadurch gekennzeichnet,**
**dass** die Herzklappenprothese (1) eine auf der Außenseite (3a) des Grundkörpers (3) angeordnete Außenschürze (11) aufweist, die den Grundkörper (3) umgibt und in der Umfangsrichtung (U) umläuft, wobei die beiden Schürzen (10, 11) eine Aufnahme (12) zum Aufnehmen von Blut ausbilden, die dazu konfiguriert ist, während der Diastole Blut aufzunehmen und anzusammeln.

2. Herzklappenprothese nach Anspruch 1, **dadurch gekennzeichnet, dass** sich die Außenschürze (11) in Richtung auf die Herzklappenprothese (1) umgebendes Gewebe (G) des Patienten ausbaucht und abdichtend an dieses anlegt, wobei das Gewebe (G) durch den Anulus (A) einer nativen Aortenklappe oder zumindest einen Abschnitt des linksventrikulären Ausflusstrakts (LVOT) gebildet ist.

3. Herzklappenprothese nach Anspruch 1 oder 2, **dadurch gekennzeichnet, dass** die Außenschürze (11) einen ersten Randbereich (11a) sowie einen zweiten Randbereich (11c) aufweist, wobei die beiden Randbereiche (11a, 11c) über einen mittleren Abschnitt (11b) der Außenschürze (11) miteinander verbunden sind.

4. Herzklappenprothese nach Anspruch 3, **dadurch gekennzeichnet, dass** der erste Randbereich (11a) der Außenschürze (11) an einem den Einströmbereich (3c) des Grundkörpers (3) begrenzenden Randbereich (31) des Grundkörpers (3) befestigt ist.

5. Herzklappenprothese nach Anspruch 3 oder 4, **dadurch gekennzeichnet, dass** der mittlere Abschnitt (11b) der Außenschürze (11) nicht oder lediglich lose am Grundkörper (3) befestigt ist.

6. Herzklappenprothese nach einem der Ansprüche 3 bis 5, **dadurch gekennzeichnet, dass** der zweite Randbereich (11c) der Außenschürze (11) am Ausströmbereich (3d) des Grundkörpers (3) befestigt ist.

7. Herzklappenprothese nach einem der Ansprüche 3 bis 6, **dadurch gekennzeichnet, dass** die Innenschürze (10) einen ersten Randbereich (10a) sowie einen dem ersten Randbereich (10a) in der axialen Richtung (z) nachfolgenden zweiten Randbereich (10c) aufweist, wobei der zweite Randbereich (11c) der Außenschürze (11) in der axialen Richtung (z) über den zweiten Randbereich (10c) der Innenschürze (10) hinaus ragt.

8. Herzklappenprothese nach Anspruch 7, wobei die Herzklappenprothese expandierten Zustand vorliegt.

9. Herzklappenprothese nach Anspruch 7 oder 8, **dadurch gekennzeichnet, dass** der zweite Randbereich (11c) der Außenschürze (11) und der zweite Randbereich (10c) der Innenschürze (10) eine in der Umfangsrichtung (U) umlaufende Einströmöffnung (O) bilden.

10. Herzklappenprothese nach einem der vorhergehenden Ansprüche, **dadurch gekennzeichnet, dass** der Grundkörper (3) expandierbar oder selbstexpandierend ist.

11. Herzklappenprothese nach einem der vorhergehenden Ansprüche, **dadurch gekennzeichnet, dass** die Herzklappenprothese (1) per Katheter implantierbar ist.

12. Herzklappenprothese nach einem der vorhergehenden Ansprüche, **dadurch gekennzeichnet, dass** die Herzklappe (2) ein biologisches Gewebe, insbesondere Perikard, aufweist.

13. Herzklappenprothese nach einem der vorhergehenden Ansprüche, **dadurch gekennzeichnet, dass** die Außenschürze (11) künstliches polymeres Material, mikrobielle Cellulose oder biologisches Gewebe umfasst oder daraus besteht.

14. Herzklappenprothese nach einem der vorhergehenden Ansprüche, **dadurch gekennzeichnet, dass** die Innenschürze (10) mikrobielle Cellulose oder biologisches Gewebe umfasst oder daraus besteht.
